Europäisches Patentamt

European Patent Office

Office européen des brevets

(19)

(11) Veröffentlichungsnummer: **0 331 973**

**A1**

(12) **EUROPÄISCHE PATENTANMELDUNG**

(21) Anmeldenummer: **89103069.4**

(22) Anmeldetag: **22.02.89**

(51) Int. Cl.⁴: **A61M 5/14 , A61M 1/02 , A61M 1/36**

(30) Priorität: **08.03.88 CH 863/88**

(43) Veröffentlichungstag der Anmeldung:
**13.09.89 Patentblatt 89/37**

(84) Benannte Vertragsstaaten:
**DE GB IT NL**

(71) Anmelder: **DOLTRON AG**
**Industriestrasse 3**
**CH-8610 Uster(CH)**

(72) Erfinder: **Manella, Paul**
**Glärnischstrasse 23**
**CH-8600 Dübendorf(CH)**

(74) Vertreter: **Blum, Rudolf Emil Ernst et al**
**c/o E. Blum & Co Patentanwälte Vorderberg 11**
**CH-8044 Zürich(CH)**

(54) Behältnis und Vorrichtung zum Erwärmen von Transfusions- oder Infusionsflüssigkeit.

(57) Das Behältnis (1) besteht aus einem Schlauch, der zu vier Schlauchspiralen (3,4,5,6) gewickelt ist und einer Halteeinrichtung (2), um die Schlauchspiralen (3, 4, 5, 6) parallel und im Abstand zueinander zu halten, so dass die einzelnen Schlauchspiralen (3,4,5,6) im wesentlichen allseitig mit einem Heizmedium zu beaufschlagen sind.

Die Vorrichtung weist zu diesem Zweck eine Warmluftquelle auf, in die das Behältnis (1) einlegbar ist. Die Temperatur der Warmluft wird über eine Messeinrichtung und eine Steuereinrichtung geregelt.

Durch die Anwendung der Warmluft wird eine effektive Erwärmung der Infusions- oder Transfusionsflüssigkeit erreicht und eine einfache Handhabung ohne tropfende Beutel ermöglicht.

Fig.1

# Behältnis und Vorrichtung zum Erwärmen von Transfusions- oder Infusionsflüssigkeit

Die vorliegende Erfindung betrifft ein Behältnis zum Erwärmen von Transfusions- oder Infusionsflüssigkeit sowie eine Vorrichtung mit einem Behältnis.

Es sind verschiedene Ausführungsformen von Bestecken und Blutwärmern bekannt, die im Aufsatz "A Review of Blood Warmers for Massive Transfusion" von W.J. Russell, Anaesthesia and Intensive Care, Vol II, No. 2, Mai 1974 beschrieben sind.

Im wesentlichen lassen sich diese Ausführungsformen in vier Gruppen unterteilen.

1. Erwärmung des gesamten Inhaltes in einem Beutel oder in einer Flasche in einem 37° warmen Wasserbad oder in einen Mikrowellenofen, der mit Mikrowellen die Flüssigkeit auf die erforderliche Temperatur bringt. Als Nachteile sind zu nennen, dass der Inhalt während der Infusion teilweise wieder abkühlt, bei notfallmässiger Verabreichung die notwendige Aufwärmzeit lange ist, bei Verwendung der Mikrowellenöfen ein immobiles und platzaufwendiges Gerät im Operationssaal vorzusehen ist, eine Temperaturkontrolle des erwärmten Inhaltes nicht möglich ist und eine Erfassung der Temperatur durch Erfahrungswerte schwierig ist.

2. Erwärmung der Infusion oder Transfusion während der Gabe durch einen durch Wasser geführtes verlängertes Schlauchstück. Diese Methode der Erwärmung während der Infusion ist weit verbreitet. Ein Schlauchstück von bis zu 10 Meter wird irgendwie aufgewickelt, durch ein Wasserbad geführt und erwärmt so die Flüssigkeit auf die erforderlichen 37° C.

Als nachteilig erweist sich hier, dass das auf 37° C erwärmte zwangsläufig offene Wasserbad starke Bakterienkulturen bildet, die im medizinischen Bereich unerwünscht sind, neben der Sterilität auch die relative Immobilität durch Ueberschwappen des Wassers beim hektischen Patiententransport ein Problem ist. Zudem ist es nicht von Vorteil, wenn man in der Nähe des Operationsfeldes mit nassen und tropfenden Einmalartikeln hantieren muss.

3. Schlauchsysteme, die um einen erwärmten Aluminiumzylinder gewickelt sind oder in eine dafür vorgesehene Nut eingefädelt werden müssen. Die Nachteile dieser Ausführungsform sind eine geringe Effektivität durch normalerweise schlechten Kontakt an relativ dickwandigen Schläuchen, die Erwärmungszeit ist dadurch relativ lang und erfordert einen sehr langen Schlauch, um auch bei schnellen Transfusionen einen thermodynamischen Schock durch Unterkühlung beim Patienten zu vermeiden.

4. Beutelsysteme, bei denen der dünnwandige Einmalbeutel mit angeschlossenem Transfusionsbesteck zwischen zwei geheizten Aluminiumplatten für eine Erwärmung der durchfliessenden Flüssigkeit sorgt. Diese Ausführungsform hat die Nachteile, dass der Einmalbeutel mit dem Transfusionsbesteck sehr teuer ist und ein grosses Volumen an Flüssigkeit benötigt. Wegen der für eine effektive Erwärmung benötigte grosse Oberfläche wird dieses System sehr voluminös, so dass in den immer zu engen Pflegebereichen um den Patienten Platzprobleme auftreten.

Zweck der Erfindung ist es, die Nachteile des Standes der Technik zu beheben.

Der Erfindung liegt die Aufgabe zugrunde, ein Behältnis zum Erwärmen von Transfusions- oder Infusionsflüssigkeit zu schaffen, mit dem durch die Aufteilung in Teilvolumen eine effektive Temperaturfeststellung und Erwärmung der Flüssigkeit durchführbar ist.

Diese Aufgabe wird erfindungsgemäss durch die Merkmale des Patentanspruches 1 gelöst.

Die mit dem Behältnis erzielbaren Vorteile sind im wesentlichen darin zu sehen, dass eine Anzahl von Teilvolumen zu einem möglichst grossen Volumen mit grosser Oberfläche auf kleinem Raum zusammengefasst sind und die Temperatur der Teilvolumen einzeln messbar sind.

Bei einem Ausführungsbeispiel ist das Behältnis ein Schlauch und die Abteile sind als Schlauchspiralen gewickelt. Daraus ergeben sich die Vorteile, dass das Behältnis in einer Mehrzahl von einzelnen jeweils zu einer Spirale zusammengefassten Teilvolumen unterteilbar ist, da der Schlauch ein standardisierter Schlauch ist für den das entsprechende Prüfzeugnis bereits vorliegt und das Behältnis somit keiner weiteren Prüfung unterzogen werden muss.

Eine Vorrichtung mit Behältnis ist erfindungsgemäss durch die Merkmale des Patentanspruches 10 gekennzeichnet.

Die mit der Vorrichtung erzielbaren Vorteile sind im wesentlichen darin zu sehen, dass die Abteile einzeln und gleichmässig mit Warmluft beaufschlagt werden und dass durch die Warmluft eine schnelle Temperaturregelung erzielt wird.

Im folgenden wird die Erfindung anhand der beiliegenden Zeichnung erläutert.

Es zeigen:

Figur 1 eine perspektivische Ansicht eines bevorzugten Ausführungsbeispiels eines erfindungsgemässen Behältnisses,

Figuren 2A - 2D eine schematische Darstellung der einzelnen Schlauchspiralen des Behältnisses gemäss Figur 1,

Figur 3 eine perspektivische Ansicht eines anderen Ausführungsbeispiels eines Behältnisses,

Figuren 4A - 4D eine schematische Darstellung der einzelnen Schlauchspiralen des Behältnisses gemäss Figur 3, -

Figur 5 eine perspektivische Ansicht eines Ausführungsbeispiels eines erfindungsgemässen Blutwärmers,

Figur 6 eine perspektivische Ansicht des in Figur 5 gezeigten Blutwärmers, welche die Rückseite des Gerätes zeigt,

Figur 7 eine perspektivische Ansicht eines Ausführungsbeispiels einer Warmluftquelle des Blutwärmers nach Figur 5,

Figur 8 eine perspektivische Ansicht eines Teils der Warmluftquelle nach Figur 7,

Figur 9 die gleiche Ansicht wie Figur 8, in der die Anordnung des Behältnisses nach Figur 1 mit Hilfe eines seiner Bestandteile dargestellt ist,

Figur 10 einen Schnitt entlang der Linie X-X in Figur 7,

Figur 11 eine Ansicht in Richtung des Pfeiles XI in Figur 9,

Figur 12 einen Schnitt entlang der Linie XII-XII in Figur 11,

Figur 13 ein Blockschema der Steuereinrichtung für den Blutwärmer nach Figur 5, und .

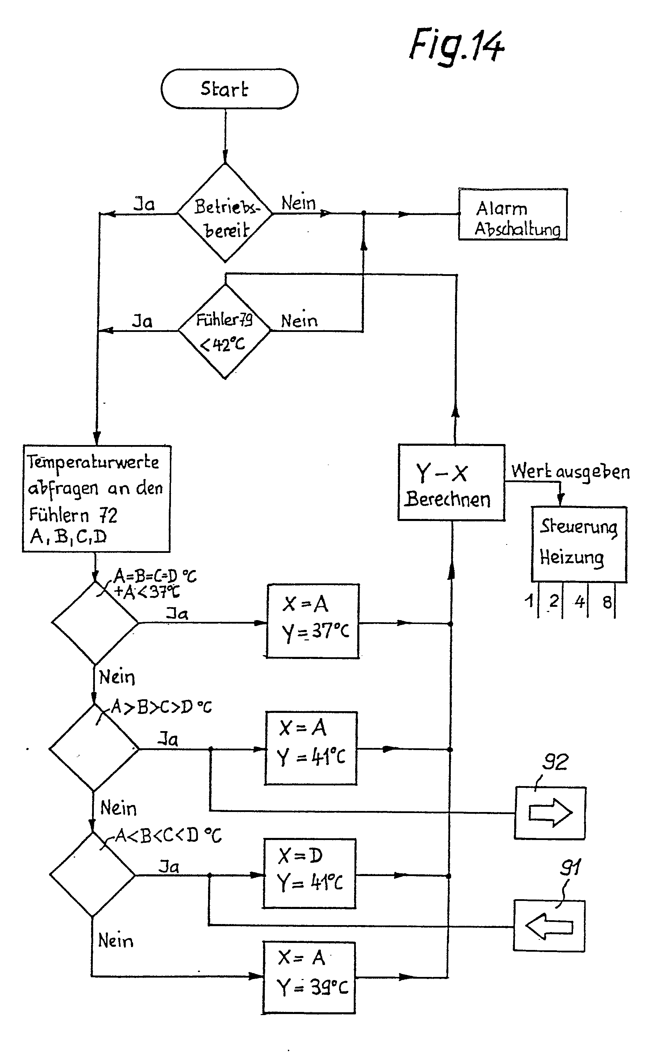

Figur 14 ein Flussdiagramm der Temperaturregelung für den Blutwärmer.

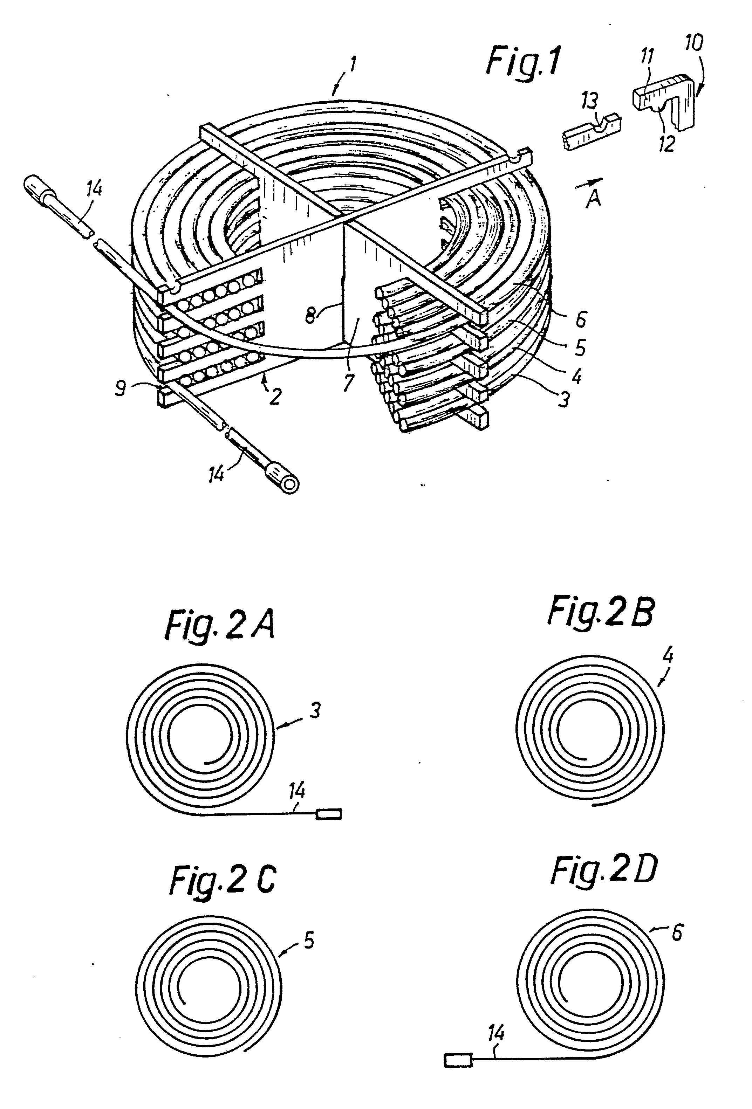

Bei dem in Figur 1 gezeigten Ausführungsbeispiel besteht das Behältnis 1 im wesentlichen aus einem Kunststoffschlauch und einer Halteeinrichtung 2. Das Behältnis 1 bildet vier Abteile, die als Schlauchspiralen 3,4,5,6 gewickelt und einzeln in der Halteeinrichtung 2 gehalten sind. Die Halteeinrichtung 2 enthält zwei längliche Platten 7, die identisch ausgebildet und zu einem Kreuz zusammengesteckt sind. Zu diesem Zweck sind die Platten 4 mit einem Schlitz 8 versehen. In den Schmalseiten der Platten 7 sind jeweils vier Schlitze 9 ausgebildet, die parallel und im Abstand zueinander in Längsrichtung der Platte 7 ausgebildet sind. Die Halteeinrichtung 2 enthält ferner vier Klammern 10, die auf die freien Enden der Platten 7 aufsetzbar sind. Die Klammer 10 ist U-förmig und weist an den Schenkeln 11 jeweils eine Wulst 12 auf, die in eine Ausnehmung 13 in der Platte 7 eingreift, um die Klammer 10 zu halten.

Um das in Figur 1 gezeigte Aufnahmegefäss 1 herzustellen, wird der Schlauch zu vier Schlauchspiralen 3,4, 5,6 entsprechend den Figuren 2A-2D gewickelt, wobei Figur 2A den Wickelsinn der Schlauchspirale 3, Figur 2B den Wikkelsinn der Schlauchspirale 4, Figur 2C den Wickelsinn der Schlauchspirale 5 und Figur 2D den Wickelsinn der Schlauchspirale 6 zeigt.

Wie aus diesen Figuren ersichtlich ist, verlaufen die Schlauchspiralen 3,4,5,6 von innen nach aussen. Während die Schlauchspirale 3 im Uhrzeigersinn gewickelt ist, sind die anderen drei Schlauchspiralen 4, 5 und 6 im entgegengesetzten Sinn gewickelt. Um dies zu erreichen, wird zuerst die Schlauchspirale 4 auf die Halteeinrichtung 2 von innen nach aussen gewickelt. Anschliessend kann die Schlauchspirale 5 gewickelt werden, indem man den Schlauch in den nächsten darüberliegenden Schlitz 9 einführt und den Schlauch von innen nach aussen auf die Halteeinrichtung 2 aufwickelt. Um die Schlauchspirale 6 zu wickeln, wird gleich wie bei der Schlauchspirale 5 vorgegangen. Abschliessend kann die Schlauchspirale 3 von innen nach aussen gewickelt werden. Bei der Wicklung wird darauf geachtet, dass zwei Schlauchabschnitte 14 für die Spender-und Empfängerseite vorhanden sind.

An den Enden der Schlauchabschnitte 14 sind die Standardanschlüsse (nicht gezeigt) für Infusions- und Transfusionsbestecke vorgesehen.

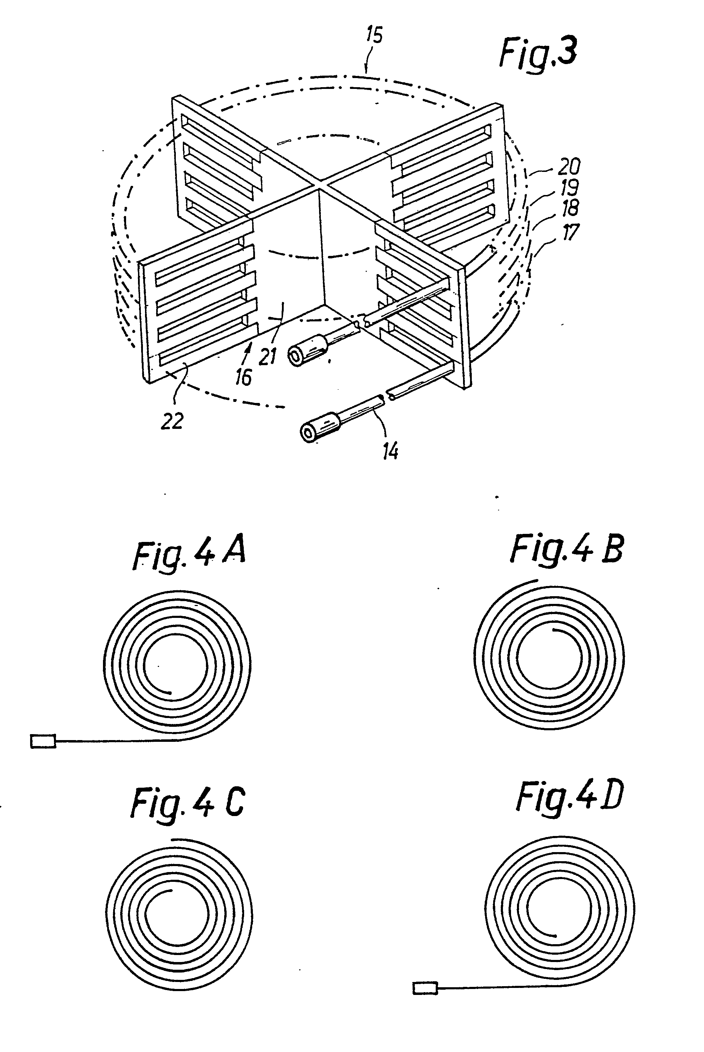

Das in Figur 3 gezeigte Behältnis 15 besteht aus vier Schlauchspiralen 17,18,19,20 und einer Halteeinrichtung 16.

Die Halteeinrichtung 16 enthält einen Stern 21 und vier Kämme 22, die auf den Stern aufsteckbar sind und die Schlauchspiralen 17 bis 20 halten.

Wie die Figuren 4A-4D zeigen, unterscheidet sich das Aufnahmebehältnis 15 von jenem in Figur 1 dargestellten dadurch, dass die Schlauchspiralen 16-20 abwechselnd von innen nach aussen gewikkelt sind.

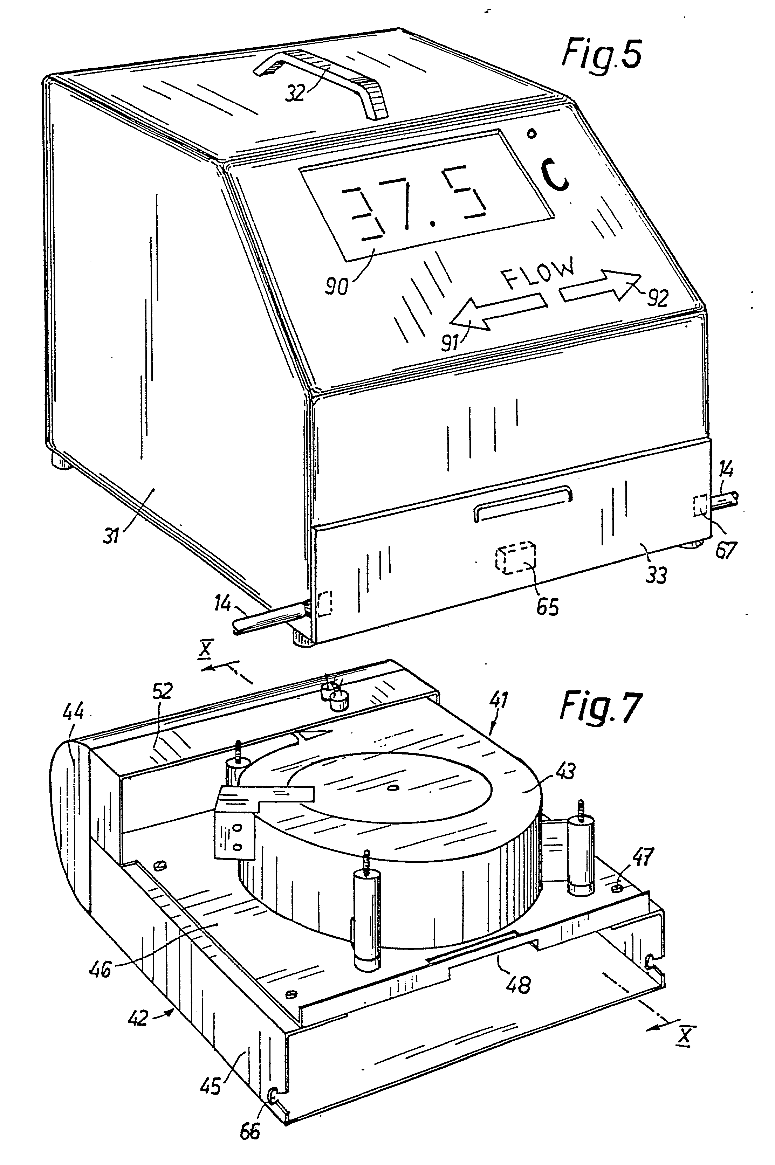

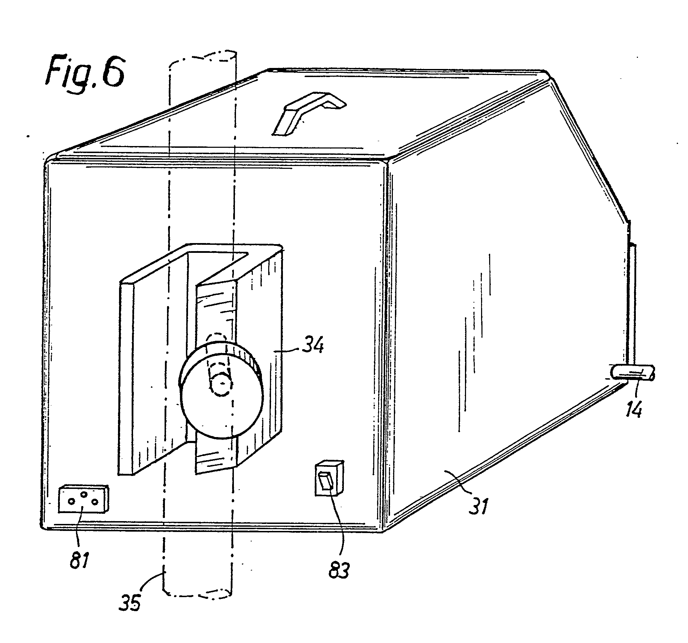

Die vorstehend beschriebenen Behältnisse werden zusammen mit einem Blutwärmer angewendet. Ein Ausführungsbeispiel eines derartigen Blutwärmers ist in den Figuren 5-13 dargestellt.

Der Blutwärmer ist als tragbares Gerät ausgebildet und hat ein Gehäuse 31 mit einem Traggriff 32. An der Frontseite sind Anzeigeinstrumente 90-92, die später beschrieben werden, und eine Klappe 33 zum Einlegen eines Behältnisses vorgesehene (Figur 5). An der Rückseite sind eine Einrichtung 34, um den Blutwärmer z.B. an einem Standrohr 35 zu befestigen, und ein Netzstecker 81 und ein Hauptschalter 83 vorgesehen, die später beschrieben werden. Ist ein Behältnis in den Blutwärmer eingelegt, sind die zwei Schlauchabschnitte 14 im vorderen Bereich des Gehäuses 31 an beiden Seiten herausgeführt.

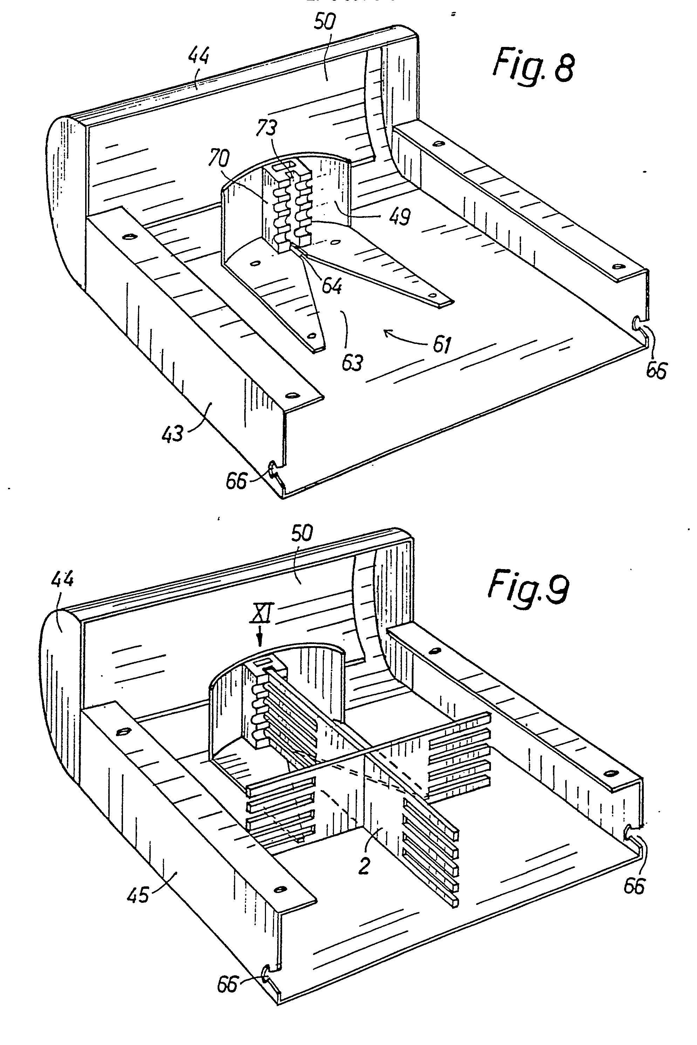

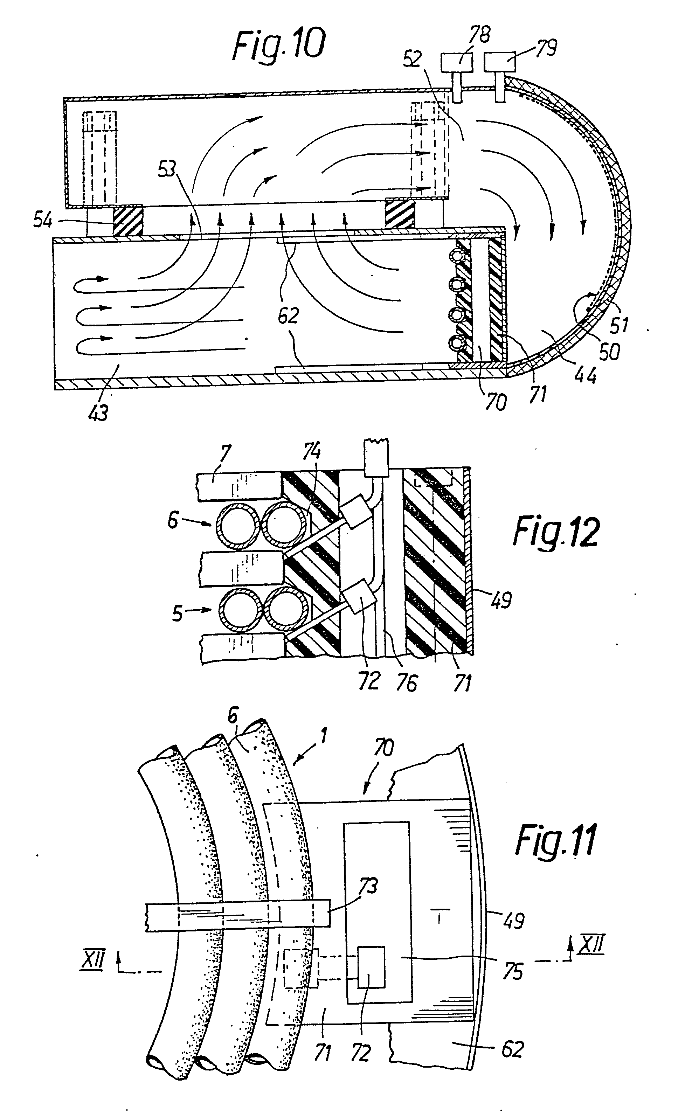

Im Gehäuse 31 sind eine Warmluftquelle 41, eine Temperatur-Messeinrichtung 70 und eine Steuereinrichtung 80 angeordnet. In Figur 7 ist eine perspektivische Ansicht und in Figur 10 ein Schnitt durch die Warmluftquelle 41 dargestellt. Diese Warmluftquelle 41 ist als Umluftofen ausgebildet und enthält ein Chassis 42, das das Aufnahmeabteil für ein Behältnis bildet, einen Ventilator 43, der die Umluft fördert, und einen Heizkanal 44, der die Luft erwärmt. Das Chassis 42 enthält einen Unterteil 45 und eine Abdeckplatte 46, die mittels Schrauben 47 auf dem Unterteil 45 befestigt ist. Die Abdeckplatte 46 bildet gleichzeitig die Schlossplatte für die Klappe 33 und ist deshalb mit einer Ausnehmung 48 versehen, die an der den Einlass für das Behältnis bildenden Seite am Chassis 42 vorgesehen ist. An der den Lufteinlass bildenden Seite ist im Chassis 42 ein Umlenkblech 49 so angeordnet, dass zwei identische Zuluftöffnungen entstehen. Der Ventilator 43 ist mit der Einlasseite auf der Abdeckplatte 46 montiert. Der Heizkanal 44 verbindet die Auslasseite des Ventilators 43 mit dem Chassis 42. Der Heizkanal 44 ist an der Innenseite mit einem Heizelement 50 und an der Aussenseite mit einer Isolationsschicht 51 versehen (Fig. 10). Wie Figur 7 zeigt, wird der Heizkanal 44 über einen Anschlussschacht 52 an den Auslass des Ventilators 43 gekoppelt. Ausserdem sind Anschlussschacht 52 und Abdeckplatte 46 miteinander verbunden. In der Abdeckplatte 46 ist ein Loch 53 vorgesehen, das dem Einlass des Ventilators 43 gegenüberliegt. Zwischen Ventilator 43 und Abdeckplatte 46 ist ferner eine Dichtung 54 montiert (Figur 10).

Wie insbesondere aus den Figuren 8 und 9 ersichtlich, ist eine Ausrichteinrichtung 60 im Chassis 42 des Umluftofens 41 vorgesehen, um ein Behältnis bezüglich dem Warmluftstrom und der Temperatur-Messeinrichtung 70 auszurichten. Die Ausrichteinrichtung 61 enthält zwei Führungsplatten 62, die einander gegenüberliegend am Unterteil 45 und an der Abdeckplatte 46 befestigt sind. Die Führungsplatten 62 haben einen V-förmigen Ausschnitt 63 und angrenzend daran einen U-förmigen Ausschnitt 64, in den die Halteeinrichtung 2 des Behältnisses (Figur 1) einführbar ist, wie Figur 9 zeigt. Die Ausrichteinrichtung 61 umfasst ferner einen Andrückteil 65, der an der Innenseite der Klappe 33 befestigt und mit der Halteeinrichtung des Behältnisses in Anlage bringbar ist, um das Behältnis in der Ausrichteinrichtung 61 zu halten. Um die zwei Schlauchabschnitte 14 aus dem Blutwärmer herausführen zu können, sind zwei Ausnehmungen 66 vorgesehen (Figur 9). Die Ausnehmungen 66 haben eine einem Schlüsselloch ähnliche Form und sind im Bereich der Frontseite im Gehäuse 31 und Chassis 45 in den Seitenwänden derselben so angeordnet, dass die Schlauchabschnitte 14 bei geöffneter Klappe 33 eingelegt werden können. An der Innenseite der Klappe 33 sind Dichtorgane 67 angeordnet, die bei geschlossener Klappe 33 die Ausnehmungen 66 abdichten.

Die Temperatur-Messeinrichtung 70 enthält einen Halter 71, der im Chassis 42 zwischen den Führungsplatten 62 angeordnet ist (Figur 10) und vier Temperaturfühler 72, an die die einzelnen Schlauchspiralen eines Behältnisses anlegbar sind. Der aus Kunststoff bestehende Halter 71 ist mit einem Schlitz 73 versehen, in den die Halteeinrichtung 2 eines Behältnisses einführbar ist (Figuren 8 und 9) und weist vier Ausnehmungen 74 auf, die übereinanderliegend so ausgebildet sind, dass ein Abschnitt der äussersten Windung der Schlauchspiralen 3,4,5,6 jeweils in eine Ausnehmung 74 hineinragt. Die Ausnehmung 74 hat einen V-förmigen Querschnitte, um sicherzustellen, dass der Schlauchabschnitt mindestens an den Wänden der Ausnehmung 74 anliegt. Für die Temperaturmessung wird dies ausgenützt, indem die Temperaturfühler 72 so in dem Halter 71 angeordnet werden, dass ein Abschnitt dieser Temperaturfühler 72 eine Wand der Ausnehmung 74 darstellt. Um die Temperaturfühler 72 mit der Steuereinrichtung verbinden zu können, ist im Halter 71 ein Loch 75 ausgebildet, durch welches die Anschlussdrähte 76 für die Temperaturfühler 72 geführt sind.

Neben den vier Temperaturfühlern 72 für das Behältnis 1 sind noch ein Temperaturfühler 78 für die Grenzwerttemperatur und ein Temperaturfühler 79 zur Ueberwachung des Ventilators 43 vorgesehen, die zwischen Ventilator 43 und Heizkanal 44 angeordnet sind (Figur 10). Ferner ist noch ein Türschalter im Bereich der Klappe 33 angeordnet, der als Sicherheitsschalter dient.

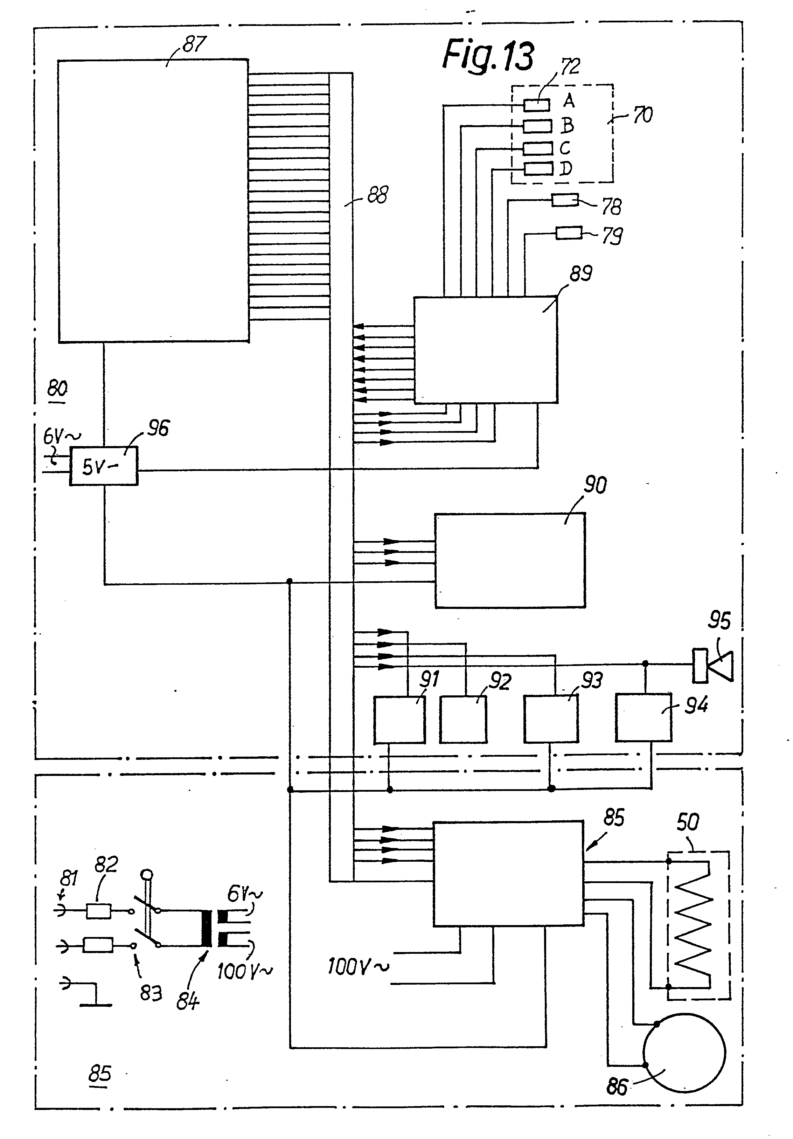

Wie erwähnt, hat der Blutwärmer eine Steuereinrichtung, die in Figur 13 dargestellt ist. Der Blutwärmer ist über einen Stecker 81 an das Netz anschliessbar. Ueber Sicherungen 82 und den Hauptschalter 84 ist der Stecker 81 mit einem Netztransformator 84 verbunden. Der Transformator 84 hat einen Abgriff für eine Spannung von 6 V~ und für eine Spannung von 100 V~ . Die Spannung von 100 V~ wird für den Leistungsteil 85 der Steuereinrichtung verwendet. Der Leistungsteil enthält die Schaltelemente zum Ein/Aus-Schalten des Heizelementes 50 und des Ventilators 86. In dem Leistungsteil 85 ist ferner ein Vollwellenunterdrückungsschaltkreis vorgesehen, der mit dem Heizelement 50 verbunden ist. Der Aufbau des Vollwellenunterdrückungsschaltkreises ist an sich bekannt und wird deshalb nicht ausführlich erläutert. Es ist aber darauf hinzuweisen, dass im vorliegenden Fall sechzehn Heizstufen, d.h. sechzehn Unterdrückungsstufen vorgesehen sind (4 Binär-Leitungen).

Das Hauptelement eines Steuerteiles der Steuereinrichtung bildet ein Mikroprozessor 87, der über einen Datenbus 88 mit einem Multiplexer 89, einem Temperaturanzeigeinstrument 90, Signallampen für den Durchflussrichtung 91,92, einer Signallampe 93 "Heizung", eines Signallampe 94 "Alarm", einen Summer 95 und dem Leistungsteil 85 verbunden ist. Neben den Temperaturfühlern 72 für die einzelnen Schlauchspiralen, und den Temperaturfühlern 78 und 79 ist auch der Türschalter 80 mit dem Multiplexer 89 verbunden. Der Steuerteil wird aus einem Gleichrichter 96 mit 5 V~ versorgt. Der Gleichrichter 96 ist an den 6V~- Abgriff des Transformators 84 angeschlossen.

Die Vier Temperaturfühler 72 sind aufgrund ihrer Anordnung von oben nach unten zusätzlich mit den Buchstaben A, B, C und D bezeichnet.

Nachfolgend wird an einem Beispiel der Temperaturregelvorgang bei einem Blutwärmer beschrieben.

Nach dem Einschalten wird die Betriebsbereitschaft des Gerätes festgestellt. Dies umfasst unter anderem die Prüfung der Temperaturfühler 72A, 72B, 72C, 72D, 78 und 79. Ist ein Temperaturfühler defekt, erfolgt die Abschaltung des Gerätes und es wird ein Alarmsignal abgegeben. Gleichzeitig mit oder anschliessend an die Feststellung der Betriebsbereitschaft wird festgestellt, ob die vom Temperaturfühler gemessens Temperatur <42°C ist. Trifft dies nicht zu, erfolgt ebenfalls die Abschaltung des Gerätes. Wird die Betriebsbereitschaft festgestellt und ist die am Temperaturfühler 79 gemessene Temperatur <42°C, werden die Temperaturwerte an den Temperaturfühler 72 abgefragt und zwar in der Reihenfolge A,B,D,C, d.h. von oben nach unten in der Temperaturmesseinrichtung 70. Werden von den Temperaturfühlern 72 die gleichen Werte gemessen und ist der am Temperaturfühler 72A gemessene Wert <37°C werden die vom Temperaturfühler 72A gemessene Temperaturwerte A als Operand X und ein vom Mikroprozessor 87 vorgegebener Solltemperaturwert 37°C als Operand Y gesetzt. Die beiden Operanden werden miteinander verknüpft und es wird ein dementsprechender Wert zur Steuerung des Heizelementes abgegeben. Beim vorstehend beschriebenen Fall handelt es sich um eine Anfahrstufe für das Gerät, in der das Behältnis vorgewärmt wird. Strömt Blut in das Behältnis ein, so werden die Temperaturfühler 72 unterschiedliche Werte messen. Wird festgestellt, dass der von dem Temperaturfühler 72A gemessene Wert A der höchste Wert ist, werden dieser Wert A als Operand X und ein vom Mikroprozessor 87 vorgegebener Solltemperaturwert 41°C als Operand Y gesetzt. Diese beiden Operanden X, Y werden verknüpft und es wird ein dementsprechender Wert zur Steuerung des Heizelementes abgegeben. Im vorstehend beschriebenen Fall strömt Blut in der Richtung von der untersten Schlauchspirale 3 zur obersten Schlauchspirale 6 durch das Behältnis (Figur 1), was durch das Anzeigeinstrument 92 angezeigt wird. Wird im Gegensatz zum vorgehend beschriebenen Fall festgestellt, dass der vom Temperaturfühler 72D gemessene Wert D der höchste Wert ist, wird analog verfahren, wobei lediglich zu berücksichtigen ist, dass das Blut in der entgegengesetzten Richtung, d.h. von der obersten Schlauchspirale 6 zur untersten Schlauchspirale 3 durch das Behältnis 1 strömt, was durch das Anzeigeinstrument 91 angezeigt wird.

Werden keine der vorstehend beschriebenen Bedingungen festgestellt, wird der vom Temperaturfühler 72A gemessene Wert als Operand X und ein vom Mikroprozessor 87 vorgegebener Solltemperaturwert 39°C als Operand Y gesetzt. Diese Operanden X, Y werden verknüpft und es wird ein dementsprechender Wert zur Steuerung des Heizelementes abgegeben. Der Vollständigkeit halber wird darauf hingewiesen, dass nach der Verknüpfung der Operanden X, Y jeweils ein Rückführungsimpuls abgegeben wird, um den Temperaturregelvorgang aufrecht zu erhalten.

## Ansprüche

1. Behältnis zum Erwärmen von Transfusions- oder Infusionsflüssigkeit, dadurch gekennzeichnet, dass es mindestens zwei Abteile bildet, welche Abteile übereinanderliegend und im Abstand zueinander so angeordnet sind, dass die Temperatur jedes Abteiles feststellbar ist und die Abteile im wesentlichen allseitig mit einem Heizmedium in Kontakt bringbar sind.

2. Behältnis nach Anspruch 1, dadurch gekennzeichnet, dass es ein Schlauch ist und die Abteile als Schlauchspiralen (3-6; 17-20) gewickelt sind.

3. Behältnis nach Anspruch 2, dadurch gekennzeichnet, dass ein Loch im Zentrum der Schlauchspiralen (3-6; 17-20) vorgesehen ist.

4. Behältnis nach Anspruch 2, dadurch gekennzeichnet, dass die Schlauchspiralen (3-6; 17-20) im gleichen Sinn gewickelt sind.

5. Behältnis nach Anspruch 2, dadurch gekennzeichnet, dass die Schlauchspiralen (3-6; 17-20) abwechselnd im gleichen und entgegengesetzten Sinn gewickelt sind.

6. Behältnis nach Anspruch 2, dadurch gekennzeichnet, dass vier Schlauchspiralen (3-6) vorgesehen sind, wobei eine Schlauchspirale (3) in einem Sinn und die anderen drei Schlauchspiralen (4-6) im entgegengesetzten Sinn gewickelt sind.

7. Behältnis nach einem der Ansprüche 2-6, dadurch gekennzeichnet, dass die benachbarten Schlauchspiralen (3-6; 17-20) untereinander befestigt sind.

8. Behältnis nach einem der Ansprüche 2-6, gekennzeichnet durch eine Halteeinrichtung (2) für die Schlauchspiralen (3-6; 17-20).

9. Behältnis nach Anspruch 8, dadurch gekennzeichnet, dass die Halteeinrichtung (2) zwei rechteckförmige Halteplatten (7) enthält, die zu einem Kreuz zusammen steckbar sind und dass die Kreuzschenkel bildenden Abschnitte mindestens zwei parallele Schlitze (9) zur Aufnahme der Schlauchspiralen (3-6) aufweist, wobei mindestens die zwei wegführenden Schlauchabschnitte (14) an den Halteplatten (7) fixiert sind.

10. Vorrichtung zum Erwärmen von Transfusions-oder Infusionsmittel mit einem Behältnis nach Anspruch 1, gekennzeichnet durch eine Warmluftquelle (41), um das Behältnis (1) mit Warmluft zu beaufschlagen, eine Temperatur-Messeinrichtung (70) mit der die Abteile des Behältnisses (1) in Kontakt bringbar ist, um die Temperatur im Behältnis (1) zu messen und eine Steuereinrichtung (80), um die Warmluftquelle (41) in Abhängigkeit von der gemessenen Temperatur zu steuern.

11. Vorrichtung nach Anspruch 10, dadurch gekennzeichnet, dass die Warmluftquelle (41) ein Chassis (42) zur Aufnahme des Behältnisses (1), einen Ventilator (43), der mit der Eintrittseite am Chassis (42) befestigt ist, und einen Heizkanal (44) mit einem Heizelement (50) aufweist, der die Austrittseite des Ventilators (43) mit dem Chassis (42) verbindet.

12. Vorrichtung nach Anspruch 11, dadurch gekennzeichnet, dass ein Umlenkblech (49) im Bereich des Lufteinlasses im Chassis (42) angeordnet ist, um den Warmluftstrom zu teilen.

13. Vorrichtung nach Anspruch 11, dadurch gekennzeichnet,dass das Chassis (42) mit einer Ausrichteinrichtung versehen ist (61), um das Behältnis (1) bezüglich dem Luftstrom und der Temperatur-Messeinrichtung (70) auszurichten, und dass eine Verschliesseinrichtung (33) vorgesehen ist, um das Behältnis (1) in der Ausrichteinrichtung (61) zu halten.

14. Vorrichtung nach Anspruch 10, dadurch gekennzeichnet, dass die Temperatur-Messeinrichtung (70) eine der Anzahl von Abteilen entsprechende Anzahl von Temperaturfühler (72) aufweist, die mit den Abteilen in Kontakt bringbar sind, um die Temperatur an jedem Abteil zu messen.

15. Vorrichtung nach Anspruch 14, wobei das Aufnahmebehältnis ein Schlauch ist und die Abteile als Schlauchspiralen (3-6; 17-20) gewickelt sind, dadurch gekennzeichnet, dass die Temperaturfühler (72) in einem Halter (71) angeordnet sind, wobei die Schlauchspiralen an die Temperaturfühler anlegbar sind, um die Temperatur an jeder Schlauchspirale (3-6; 17-20) zu messen.

16. Vorrichtung nach Anspruch 15, dadurch gekennzeichnet, dass die Temperaturfühler (72) mit einer Schaltungseinheit (89) verbunden sind, um die graduelle Temperaturänderung über die durch die Schlauchspiralen gebildeten Abteile zu messen.

17. Vorrichtung nach Anspruch 10, dadurch gekennzeichnet, dass Heizelemente (50) mit einem Vollwellenunterdrückungsschaltkreis (85) verbunden sind, um die Heizleistung in Abhängigkeit der gemessenen Temperatur zu steuern.

EP 0 331 973 A1

*Fig.1*

*Fig.2A*

*Fig.2B*

*Fig.2C*

*Fig.2D*

**Fig.3**

**Fig.4 A**

**Fig.4 B**

**Fig.4 C**

**Fig.4 D**

*Fig.5*

*Fig.7*

Fig.6

EP 0 331 973 A1

Fig. 8

Fig. 9

*Fig.10*

*Fig.12*

*Fig.11*

Fig.13

## Fig.14

```
                    ┌─────────┐
                    │  Start  │
                    └────┬────┘
                         │
              ◇ Betriebs-bereit ◇
         Ja ←───        ───→ Nein ──→ ┌──────────────┐
                                      │    Alarm     │
                                      │ Abschaltung  │
                                      └──────────────┘
```

Betriebs-bereit — Ja / Nein

Fühler 79 < 42°C — Ja / Nein

Alarm Abschaltung

Temperaturwerte abfragen an den Fühlern 72 A, B, C, D

Y - X Berechnen

Wert ausgeben

Steuerung Heizung

| 1 | 2 | 4 | 8 |
|---|---|---|---|

A = B = C = D °C
+ A < 37°C — Ja → X = A  Y = 37°C

Nein

A > B > C > D °C — Ja → X = A  Y = 41°C

Nein

A < B < C < D °C — Ja → X = D  Y = 41°C

Nein

X = A  Y = 39°C

92

91

| Kategorie | EINSCHLÄGIGE DOKUMENTE | | EP 89103069.4 |
|---|---|---|---|
| | Kennzeichnung des Dokuments mit Angabe, soweit erforderlich, der maßgeblichen Teile | Betrifft Anspruch | KLASSIFIKATION DER ANMELDUNG (Int. Cl.4) |
| D,A | ANAESTHESIA AND INTENSIVE CARE, Band II, Nr. 2, Mai 1974<br><br>W. J. RUSSELL: "A Review of Blood Warmers for Intensive Care." Seiten 109-130<br><br>+ Seiten 114-119 +<br><br>-- | 1 | A 61 M 5/14<br>A 61 M 1/02<br>A 61 M 1/36 |
| A | DE - A1 - 3 430 534 (W. WEIPKEMA)<br><br>+ Fig. 1,2,4; Patentansprüche 1,4,5,24,25,35-49,55 +<br><br>---- | 1 | |
| | | | **RECHERCHIERTE SACHGEBIETE (Int. Cl.4)**<br><br>A 61 M 1/00<br>A 61 M 5/00 |
| Der vorliegende Recherchenbericht wurde für alle Patentansprüche erstellt. | | | |

| Recherchenort | Abschlußdatum der Recherche | Prüfer |
|---|---|---|
| WIEN | 20-06-1989 | LUDWIG |